# EUROPEAN PATENT SPECIFICATION

(11) **EP 0 274 198 B1**
(45) Date of publication and mention of the grant of the patent: **21.04.1993**
(21) Application number: 87309911.3
(22) Date of filing: 10.11.1987
(51) Int. Cl.: G01N 33/543, G01N 33/76

(54) **Immunoassay kit**
Immunologischer Testkit
Kit d'essai immunologique

(30) Priority: 10.11.1986 GB 8626767
(43) Date of publication of application: 13.07.1988
(73) Proprietor: UNILEVER PLC, London EC4P 4BQ (GB); UNILEVER N.V., 3013 AL Rotterdam (NL)
(72) Inventor: Coley, John, Leicester LE2 3PR (GB); Gani, Mohamed Mutwahar, Felmersham Bedfordshire, MK43 7JE (GB)
(74) Representative: Butler, David John

(56) References cited:
- EP-A- 0 034 050
- EP-A- 0 096 463
- EP-A- 0 151 320

## Description

The present invention relates to assays utilising specific binding, and particularly to immunoassays.

More particularly, the invention relates to solid-phase immunoassays which utilise labelling compounds, such as enzymes, linked to antibodies.

Labelling compounds, such as enzymes, tend to dissociate from the antibody and may therefore become free in the sample. Furthermore, certain enzymes which are very suitable as labelling compounds, e.g. alkaline phosphatase, occur naturally in many biological samples to which the invention may be applied. The presence of such free labelling compounds can lead to spurious or inaccurate analytical results.

The invention provides an assay kit comprising:
a) a reagent comprising a bi-functional antibody having a first specificity for an analyte to be assayed and a second different specificity for a labelling compound;
b) the labelling compound; and
c) solid phase, to which are bound molecules of the analyte and/or a cross-reactive analogue of the analyte, and a specific binding partner having specificity, with respect to the labelling compound, identical to the specificity of the bi-functional antibody.

Such a kit can be used in an assay procedure comprising the steps of:
i) contacting the antibody-containing reagent with a sample which may contain the analyte;
ii) following an appropriate incubation period (if any), contacting the sample with the solid phase;
iii) separating the sample from the solid phase; and
iv) analysing either (or both of) the separated sample and the solid phase for the presence of the label, from which analysis the presence or otherwise of the analyte in the original sample can be inferred.

The invention involves contacting the sample with solid phase to which is coupled a specific binding partner for the labelling compound, which specific binding partner is identical to that comprising the specific binding site on the antibody. Any free labelling compound will therefore become bound to this solid phase and will not interfere with the analytical result.

The antibody in the reagent is a so-called bi-functional or "double-headed" antibody. Such antibodies have two specific binding sites which have specificities for different molecules. Such antibodies can be synthesised chemically, e.g. by modification of monoclonal antibodies. Alternatively, cell-lines producing monoclonal antibodies for two different molecules (i.e. the analyte and the label) can be fused and selected for hybrid lines which produce antibodies having the desired dual specificity. EP-A1-0096463 describes bi-functional antibodies and their general use in immunoassays, but does not suggest their use in an assay kit format as claimed herein.

The solid phase with which the incubated sample is contacted can be in any physical form, e.g. the surface of a vessel, a strip or sheet or a peg which can be dipped into the sample. In one embodiment of the invention, the solid phase is a particulate solid of which can be added to the sample, and the subsequent separation of the solid phase from the sample can be achieved by, for example, filtration or decantation. In a particularly preferred embodiment of the invention, the solid phase is located in a column through which the sample can be eluted. There should be excess analyte/analogue on the solid phase to ensure that all of the (unlabelled) antibody is bound.

The label can be any compound the presence of which can be easily monitored, e.g. by means of chemiluminescence, fluorescence, radioactivity or colour. Enzymes are already widely used as labelling compounds in immunoassays and are very suitable for this use in the context of the present invention. The presence of the enzyme-linked antibody in the separated sample can be determined by the addition of appropriate reagents which participate in a chemical reaction which the enzyme catalyses. Preferred chemical reactions are ones in which a readily discernable phenomenon is produced, such as a colour change or the production of a chemiluminescence.

A preferred embodiment of the invention is an assay utilising a column which contains a single phase, or a combination of two solid phases, which (jointly) carry molecules of the analyte to be determined (or analogue) and antibodies specific to the labelling compound.

The following detailed descriptions, which is given by way of example only, illustrates how the benefits of the invention can be obtained.

### Example 1: Preparation of Bifunctional Antibodies

### A. Preparation of Parental Hybridoma Cell Lines

Both parental lines were derived by mouse spleen myeloma fusions. The basic fusion protocol was as described in European Patent specification No. 0151320. One line expressed antibodies against alkaline phosphatase and the other against human chorionic gonadotrophin (HCG).

### a) Preparation of Primed Splenocytes

Balb/c mice are immunised intraperitioneally with the appropriate antigen (HCG or Alkaline phosphatase) followed by booster immunisations on days 42, 54 and 61. They also received intravenously a booster 3 days before cell fusion. The mice were sacrified and spleen cells prepared aseptically by removing the spleen and teasing the cells into saline. The cell suspension was then centrifuged at 200 x g for 5 min, and the pellet resuspended in saline at 10⁷ cells per ml. These steps were carried out at room temperature.

### b) Preparation of Myeloma Cells for Fusion

Balb/c myeloma cells (NS1) deficient in hypoxanthine guanine phosphoribosyl transferase (HGPRT) were maintained on Delbeco's modified medium (DMEM) containing 10% foetal calf serum and 10% horse serum. The growth of the line was inhibited by selective hypoxanthine aminopterine thymidine medium (HAT). On the day of fusion, the myeloma cell suspensions were centrifuged at 200 x g for 5 min, the pellet resuspended in saline, centrifuged for 5 min at 200 x g and finally suspended in saline at a concentration of 10⁷ cells/ml.

### c) Preparation of Peritoneal Macrophages

On the day before fusion 2-3 adult Balb/c mice were killed, the abdominal skin removed and 4-5ml saline injected peritoneally, entering directly above the symphysis and letting the tip of the needle rest over the right lobe of the liver. After gentle massage of the abdomen the fluid is withdrawn, yielding 1-3 x 10⁶ macrophages per mouse. The cells were collected in polypropylene tubes, washed with the DMEM, pooled and counted, then centrifuged for 5 min at 200 x g and re-suspended in HAT at 5 x 10⁵ cells/ml. The cells were then distributed at 2-3 x 10⁴ cells per cup in Linbro tissue culture plates (Flow Laboratories) and trays left in a CO₂ incubator ready for use next day.

### d) Fusion

For fusion, 2 x 10⁷ spleen cells were combined with 5 x 10⁷ myeloma cells and the suspension centrifuged at 200 x g for 5 minutes. The supernatant was discarded and the pellet loosened. Then to the pellet 1.0ml of 50% solution (w/v) of polyethylene glycol (PEG) 3000 or 0.2ml of 35% solution (w/v) of PEG 1500 was added. The cells were incubated for 1 minute, under constant agitation at room temperature followed by immersion for 2 minutes, without agitation in a 37°C water bath. The fusion was stopped by slowly adding 20ml saline over the next 5 minutes. The cells were centrifuged for 5 minutes at 200 x g. The supernatant was discarded and the pellet gently resuspended in HAT. The cells were then distributed at 7 x 10⁴ (spleen) concentration per cup in the pre-treated Linbro plates. The plates were incubated at 37°C in a 6% CO₂ incubator.

### e) Maintenance

Cultures were inspected on days 4, 7, 10 and then every other day, up to the end of the third week. On each of these days, 1ml of medium was removed by suction and replaced by fresh HAT medium up to day 21, and then by normal growth medium thereafter. The supernatant from wells containing more than 10⁴ hybrid cells was tested for antibodies to the alkaline phosphatase or HCG, as appropriate, using an enzyme-linked immunoassay. The positive clones were then transferred to a 25cc flask containing 2ml fresh medium. As soon as the hybrids had grown almost to confluence in the 25cc flasks, the cells were preserved by being frozen in 10% DMSO.

### B) Preparation of Quadroma Cell Line

### a) Universal Fusion Partners (UFP)

The UFP was derived from a fusion between a Balb/c mouse (hyperimmunised with alkaline phosphate) and NS1-myeloma cells (Flow Laboratories). This was subjected to 8-azaguanine and ouabain treatment.

The selection for 8-azaguanine involved a process of adaptive growth in gradually increasing concentrations of the drug beginning with 10µg/ml. Cells were exposed to this concentration for a few generations. The process is repeated with increasing concentration of 8-azaguanine until healthy viable cells are maintained in the presence of 150µg/ml of the drug. These drug treated cells lack functional HPRT activity and are therefore HAT sensitive. The 8-azaguanine resistant cell lines, are then exposed to the second drug ouabain (0.1mM to 2mM) by a similar adaptive growth procedure.

### b) Fusion

Approximately 2 x 10⁷ cells of each parent were washed three times in PBS glucose and mixed together. The mixed cell suspension was centrifuged at 1800rpm for 30 seconds. The supernatant was removed and the pellet gently agitated to form a thick slurry of loose cells. 1ml of 50% PEG 3000 (w/v) pH 7.8 in PBS glucose was slowly added to the cell slurry. Cells were spun at 800rpm for 3 minutes and the supernatant was removed after a contact time of 8 minutes. The pellet was resuspended in 20ml PBS glucose and spun for a further 3 minutes at 800 RPM. A selective medium (DMEM + 10% horse serum + HAT + 0.3mM ouabain) was added to the washed pellet and the cells dispersed into Limbro plates and incubated in a CO₂ incubator at 37°C. The selective medium kills both of the parental hybridomas while quadromas, which have retained functional HPRT and the ouabain resistant mutation, survive and grow.

Quadromas expressing antibodies which bind simultaneously to both desired specificities, are cloned using the limited dilution technique. Single clones which were stable and active for bifunctionality were cultured to produce large amounts of antibodies either in vivo as ascites or in vitro in serum free medium.

### c) Maintenance

Cultures were inspected on days 4, 7, 10 and then every other day, up to the end of the third week. On each of these days, 1ml of medium was removed by suction and replaced by fresh selective medium up to day 21 and then by normal growth medium thereafter. The supernatant from wells containing more than 10⁴ hybrid cells was tested for antibodies to alkaline phosphatase and HCG using an enzyme linked immunoassay. The positive clones were then transferred to a 25cm² flask containing 2ml fresh medium.

As soon as the hybrids had grown to almost confluence in the 25ml flasks, the cells were frozen in the presence of 10% DMSO, and to produce antibodies in vivo cells were injected into pristane-treated mice. Ascitic fluid was collected from these mice after 15 days.

### Purification

Bifunction antibodies were purified by two stage affinity chromatography. The first column contained HCG coupled to CNBr-activated Sepharose® 4B (Pharmacia/Sweden) using conventional procedures. The second was packed with alkaline phosphatase coupled to CNBr-activated Sepharose®. In the case of the HCG column, the antibody was eluted using 4m Mg Cl₂ and for the alkaline phosphatase the antibody was eluted with 50% ethylene glycol.

### Cell Line Deposition

A sample of a quadroma cell line, made according to the foregoing procedure, expressing a bifunctional antibody having specifities for alkaline phosphatase enzyme and HCG has been deposited, in accordance with the provisions of the Budapest Treaty, with the European Collection of Animal Cell Cultures (PHLS Centre for Applied Microbiology & Research, Porton Down, Salisbury, Wiltshire SP4 OJG, UK) and accorded deposit reference number 86121501.

The bifunctional antibody expressed by the deposited cell line was used in Examples 2 and 3 below.

### Example 2

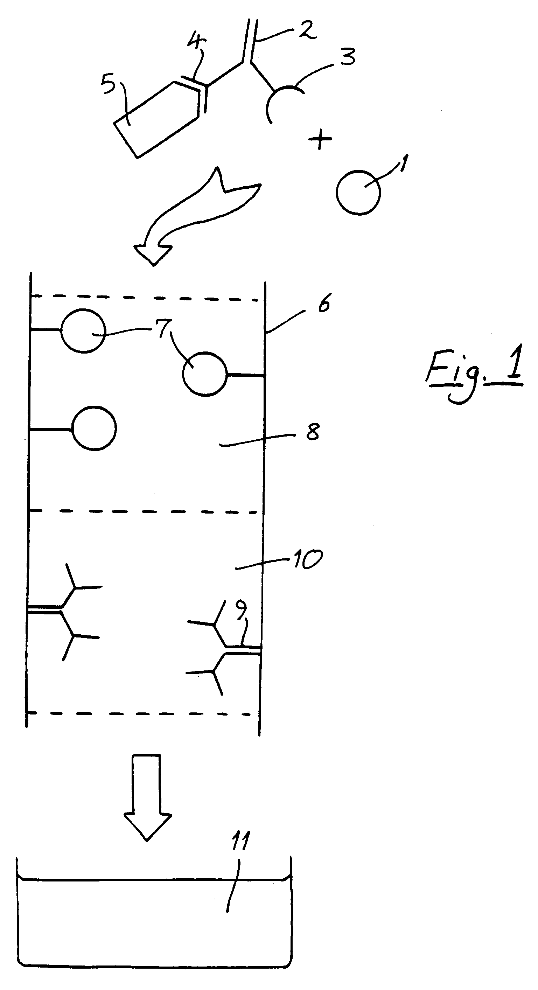

The accompanying drawing (Figure 1), illustrates an analytical procedure in accordance with the invention.

A test sample, which may contain HCG (1), was incubated with a bifunctional antibody (2) having specificities (3,4) for HCG and also to alkaline phosphatase (5) to which the antibody had previously been coupled. The incubated sample was eluted in a column (6) to which was bound HCG (7) in an upper zone (8), and anti-alkaline phosphatase antibodies (9) in a lower zone (10). The anti-alkaline phosphatase antibodies were derived from the same parent cell line from which the bifunctional antibody had been prepared. The eluted sample (11) was tested for the presence of alkaline phosphatase enzyme. An HCG positive sample gave a positive result for the enzyme via a colour change, whereas a negative sample gave no colour change.

The method of the invention, can be applied to analytes of any molecular size, e.g. haptens and proteins.

The bound components on the solid phase, i.e. the analyte and the antibody to the enzyme must be present in excess over the liquid phase reagents. There must be sufficient analyte to bind all of the bifunctional reagent, and also sufficient antibody to the alkaline phosphatase to bind all of the free enzyme present, including enzyme naturally occurring in the test sample.

The anti-alkaline phosphatase antibody bound to the solid phase is preferably against the same enzyme isotype, i.e. it must have the same specificity as that in the bifunctional antibody. This will prevent the enzyme from binding simultaneously to the bifunctional antibody, and to the solid phase antibody. Haptens can be coupled directly to the solid phase e.g. AH-Sepharose®, or indirectly via a carrier molecule such as bovine serum albumin to CNBr Sepharose®. Protein antigens can be coupled directly to the solid phase.

The assay format is a single step, and does not require any washing steps. The assay procedure can be conducted rapidly e.g. in 1-2 minutes.

The assay format can generate a positive colour response for a positive level of analyte. This is particularly useful for monitoring hormone levels (e.g. E3G or P3G) progressively during the menstrual cycle, thus giving a clearer picture of events taking place. An immunoassay of the invention also has the advantage of producing a signal by means of a single antibody, whereas other assay formats e.g. "sandwich EIA" require two antibodies. The need to find antibodies which pair together in the "sandwich" format is thus avoided.

### Assay Protocol

A column (5cm x 3cm) was prepared using HCG coupled Sepharose® 4B and anti-alkaline phosphatase monoclonal antibody coupled to Sepharose® 4B.

The bifunctional reagent in 0.5ml of phosphate buffered saline (PBS) containing 2µg/ml of bifunctional antibody and 10µg/ml alkaline phosphatase enzyme was mixed with sample containing HCG (10µl). PBS was used as a negative control.

The reagent mixture was loaded onto the column which was then eluted with PBS and the eluate allowed to run into a substrate solution comprising p-nitrophenyl phosphate (1mg/ml) in 1M diethanolamine buffer at pH 9.8. Formation of a yellow colour within 1-2 minutes indicated a positive reaction to the presence of HCG in the sample.

### Example 3

This example illustrates a further analytical procedure in accordance with the invention. In this instance a colour is produced on the solid phase and is indicative of a negative level of analyte in the sample.

The assay format involves 3 manipulations:
a) addition of sample and bifunctional reagent to the column;
b) washing with buffer to remove free bifunctional reagent and free enzyme; and
c) addition of substrate.

The column contains only HCG (analyte) bound to Sepharose®.

As in the previous example, the total assay time can be very short, e.g. 1-2 minutes.

### Protocol

The bifunctional antibody reagent was prepared in PBSTA (0.01M phosphate buffered saline pH 7.3 containing 0.15% surface active agent (Tween 20) and 0.01% sodium azide). The antibody concentration was 2.0µg/ml, and the enzyme concentration was 10µg/ml.

To test for the presence of HCG in a sample, 0.5ml of bifunctional reagent was mixed with 0.5ml PBSTA containing 20IU HCG (positive sample). A similar mixture, but with the HCG omitted, was used as a negative control.

200µl of the reaction mixture was immediately loaded onto the column which contained 500µl of packed sepharose 4B HCG gel.

As soon as the reaction mixture had penetrated the gel, the column was washed with 0.5ml buffer (PBSTA) followed by 200µl substrate solution (p-nitrophenyl phosphate 1mg per ml in diethanolamine buffer, pH 9.8).

Within 1 minute of addition of the substrate solution, a yellow colour developed in the column in the case of the negative sample, whereas in the case of the HCG containing sample, the column remained colourless.

### Example 4

In a further embodiment, the bifunctional antibody reagent is contained in a layer dried in the upper regions of a column with the solid phase to which the analyte is bound, being in the lower regions of the column. The contents of the lower regions of the column, can have the same configuration as described above in Examples 2 and 3.

The addition of sample, e.g. urine, rehydrates the bifunctional reagent at the top of the column. The bifunctional reagent can be dried in a sucrose layer for example.

## Claims

1. An assay kit comprising:
a) a reagent comprising a bi-functional antibody having a first specificity for an analyte to be assayed and a second different specificity for a labelling compound;
b) the labelling compound; and
c) solid phase, to which are bound molecules of the analyte and/or a cross-reactive analogue of the analyte, and a specific binding partner having specificity, with respect to the labelling compound, identical to the specificity of the bi-functional antibody.

2. An assay kit according to claim 1, wherein the solid phase is located in a column through which an assay sample can be eluted.

3. An assay kit according to claim 1, wherein the solid phase is a strip or sheet.

4. An assay kit according to any one of claims 1 to 3, wherein the labelling compound is an enzyme.

5. An assay kit according to any one of the preceding claims, wherein the solid phase comprises a combination of two solid phases which jointly carry the analyte (or analogue) and the specific binding partner of the labelling compound.

6. An assay kit according to any one of the preceding claims, wherein the analyte (or analogue) and the specific binding partner for the labelling compound are carried on different zones of the solid phase.

## Patentansprüche

1. Testkit, umfassend
a) ein Reagenz, umfassend einen bifunktionalen Antikörper mit einer ersten Spezifität für einen zu bestimmenden Analyten und einer zweiten unterschiedlichen Spezifität für eine Markierungsverbindung,
b) die Markierungsverbindung und
c) eine Festphase, an die Moleküle des Analyten und/oder eines kreuzreagierenden Analogs des Analyten und ein spezifischer Bindungspartner mit einer Spezifität bezüglich der Markierungsverbindung, die zur Spezifität des bifunktionalen Antikörpers identisch ist, gebunden sind.

2. Testkit nach Anspruch 1, worin sich die Festphase in einer Säule befindet, durch welche die Testprobe eluiert werden kann.

3. Testkit nach Anspruch 1, worin die Festphase ein Streifen oder eine Folie ist.

4. Testkit nach irgendeinem der Ansprüche bis 1 bis 3, worin die Markierungsverbindung ein Enzym ist.

5. Testkit nach irgendeinem der vorhergehenden Ansprüche, worin die Festphase eine Kombination aus zwei Festphasen umfaßt, die zusammen den Analyten (oder das Analog) und den spezifischen Bindungspartner der Markierungsverbindung tragen.

6. Testkit nach irgendeinem der vorhergehenden Anspruche, worin der Analyt (oder das Analog) und der spezifische Bindungspartner für die Markierungsverbindung auf verschiedenen Abschnitten der Festphase enthalten sind.

## Revendications

1. Un kit de dosage comprenant :
a) un réactif comprenant un anticorps bi-fonctionnel ayant une première spécificité pour un analyte à tester et une seconde spécificité différente pour un composé de marquage ;
b) le composé de marquage ; et
c) une phase solide, à laquelle sont liées des molécules de l'analyte et/ou un analogue réactionnel croisé de l'analyte, et un partenaire de liaison spécifique ayant une spécificité, en ce qui concerne le composé de marquage, identique à la spécificité de l'anticorps bi-fonctionnel.

2. Un kit de dosage selon la revendication 1, dans lequel la phase solide est située dans une colonne à travers laquelle un échantillon à doser peut être élué.

3. Un kit de dosage selon la revendication 1, dans lequel la phase solide est une bandelette ou une feuille.

4. Un kit de dosage selon l'une des revendications 1 à 3, dans lequel le composé de marqage est une enzyme.

5. Un kit de dosageselon l'une quelconque des revendications précédentes, dans lequel la phase solide comprend une combinaison de deux phases solides qui, ensemble, portent l'analyte (ou un analogue) et le partenaire de liaison spécifique du composé de marquage.

6. Un kit de dosage selon l'une quelconque des revendications précédentes, dans lequel l'analyte (ou analogue) et le partenaire de liaison spécifique du composé de marquage sont portés par différentes zones de la phase solide.
